(19) Europäisches Patentamt
European Patent Office
Office européen des brevets

(11) EP 2 955 191 B1

(12) EUROPEAN PATENT SPECIFICATION

(45) Date of publication and mention of the grant of the patent:
**22.02.2017 Bulletin 2017/08**

(51) Int Cl.:
***C07J 9/00*** *(2006.01)* ***A61K 31/575*** *(2006.01)*
***A61P 13/12*** *(2006.01)*

(21) Application number: **14171682.9**

(22) Date of filing: **09.06.2014**

(54) **Compound, composition and method for treating and/or ameoliorating kidney disease**

Verbindung, Zusammensetzung und Verfahren zur Behandlung und/oder Verbesserung einer Nierenerkrankung

Composé, composition et procédé pour traiter et/ou améliorer une maladie rénale

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.12.2015 Bulletin 2015/51**

(73) Proprietor: **Trineo Biotechnology Co. Ltd**
**New Taipei City 221 (TW)**

(72) Inventors:
- **Huang, Cheng-Po**
  **300 Hsinchu City (TW)**
- **Chen, Teng-Hai**
  **733 Tainan (TW)**
- **Chen, Kuang Dee**
  **600 Chiayi City (TW)**
- **Su, Chun-Tao**
  **103 Taipei City (TW)**

(74) Representative: **Lang, Christian**
**LangPatent Anwaltskanzlei**
**IP Law Firm**
**Rosenheimer Straße 139**
**81671 München (DE)**

(56) References cited:
- **PILLAI T G ET AL: "Prevention of cisplatin induced nephrotoxicity by terpenes isolated from Ganoderma lucidum occurring in Southern Parts of India", EXPERIMENTAL AND TOXICOLOGIC PATHOLOGY, JENA, DE, vol. 63, no. 1-2, 1 January 2011 (2011-01-01), pages 157-160, XP027561282, ISSN: 0940-2993 [retrieved on 2009-12-03]**
- **DATABASE CA [Online] CHEMICAL ABSTRACTS SERVICE, COLUMBUS, OHIO, US; PILLAI, THULASI G. ET AL: "Protective effect of terpenes isolated from Ganoderma", XP002731252, retrieved from STN Database accession no. 2007:598906 & PILLAI, THULASI G. ET AL: "Protective effect of terpenes isolated from Ganoderma", AMALA RESEARCH BULLETIN , 26, 152-158 CODEN: ARBMCS; ISSN: 0971-4987, 2006,**
- **Toshihiro Akihisa ET AL: "Anti-inflammatory and anti-tumor-promoting effects of triterpene acids and sterols from the fungus Ganoderma lucidum", Chemistry & biodiversity, vol. 4 1 February 2007 (2007-02-01), pages 224-231, XP55146988, Switzerland Retrieved from the Internet: URL:http://www.ncbi.nlm.nih.gov/pubmed/173 11233**
- **NGUYEN THE TUNG ET AL: "Inhibitory effect on NO production of triterpenes from the fruiting bodies of Ganoderma lucidum", BIOORGANIC & MEDICINAL CHEMISTRY LETTERS, vol. 23, no. 5, 1 March 2013 (2013-03-01), pages 1428-1432, XP55146953, ISSN: 0960-894X, DOI: 10.1016/j.bmcl.2012.12.066**
- **KENJI IWATSUKI ET AL: "Lucidenic Acids P and Q, Methyl Lucidenate P, and Other Triterpenoids from the Fungus Ganoderma lucidum and Their Inhibitory Effects on Epstein-Barr Virus Activation", JOURNAL OF NATURAL PRODUCTS, vol. 66, no. 12, 1 December 2003 (2003-12-01), pages 1582-1585, XP55146952, ISSN: 0163-3864, DOI: 10.1021/np0302293**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 955 191 B1

- QING-XI YUE ET AL: "Interaction of Ganoderma triterpenes with doxorubicin and proteomic characterization of the possible molecular targets of Ganoderma triterpenes", CANCER SCIENCE, vol. 99, no. 7, 1 July 2008 (2008-07-01), pages 1461-1470, XP55146987, ISSN: 1347-9032, DOI: 10.1111/j.1349-7006.2008.00824.x
- REEM N. EL-NAGA: "Pre-treatment with cardamonin protects against cisplatin-induced nephrotoxicity in rats: Impact on NOX-1, inflammation and apoptosis", TOXICOLOGY AND APPLIED PHARMACOLOGY, vol. 274, no. 1, 1 January 2014 (2014-01-01), pages 87-95, XP055147082, ISSN: 0041-008X, DOI: 10.1016/j.taap.2013.10.031
- DOMITROVIC ROBERT ET AL: "Berberine exerts nephroprotective effect against cisplatin-induced kidney damage through inhibition of oxidative/nitrosative stress, inflammation, autophagy and apoptosis", FOOD AND CHEMICAL TOXICOLOGY, vol. 62, 8 September 2013 (2013-09-08), pages 397-406, XP028783558, ISSN: 0278-6915, DOI: 10.1016/J.FCT.2013.09.003

## Description

## BACKGROUND OF THE INVENTION

1. FIELD OF THE INVENTION

The present disclosure relates to methods and compositions for the treatment or prophylaxis of kidney diseases, including acute kidney injury (AKI) and chronic kidney disease (CKD).

2. DESCRIPTION OF RELATED ART

Acute kidney injury (AKI) or acute renal failure is a syndrome characterized by the rapid loss of the kidney's excretory function and is typically diagnosed by the accumulation of end products of nitrogen metabolism (urea and creatinine) or decreased urine output, or both. It is the clinical manifestation of disorders such as high blood pressure and diabetes that affect the kidney acutely. AKI is common in hospital patients and very common in critically ill patients. Causes of AKI can include, but are not limited to, ischemia/reperfusion, trauma, kidney disease, and kidney transplantation. No specific therapies have emerged that can attenuate AKI or expedite its recovery; thus, most treatment is purely supportive. If patients survive their illness and do not have premorbid chronic kidney disease (CKD), they typically recover to dialysis independence. However, evidence suggests that patients who have had acute kidney injury are at increased risk of subsequent chronic kidney disease.

Exp. Toxicol. Pathol. Vol 63, pp 157-160 (2011) and Amala Res. Bull. Vol 26, pp 152-158 (2006) both disclose the use of a triterpene fraction from *Ganoderma lucidum* in the treatment of kidney disease.

It is believed that glomerular filtration rate (GFR) is the most relevant metric for determining the extent of AKI and progression of CKD. Reduction in the GFR secondary to kidney injury, either acute or chronic, are accompanied by increases in blood urea nitrogen (BUN) and serum creatinine levels. Thus, if a compound or a composition capable of increasing GFR while reducing or suppressing BUN and serum creatinine levels, such compound or composition would be a potential lead compound or composition for the manufacture of a medicament for the treatment or prophylaxis of kidney disease, including AKI and CKD.

## SUMMARY

The present disclosure is based, at least in part, the unexpected discovery that a triterpenoid isolated from *Ganoderma lucidum,* and a composition comprising the same, are capable of suppressing BUN and serum creatinine levels, as well as increasing GFR; thus compositions comprising this triterpenoid as defined herein are provided for the treatment or prophylaxis of kidney disease, including AKI and CKD.
Accordingly, there is provided triterpenoid isolated from *Ganoderma lucidum* having the structure of formula (I),

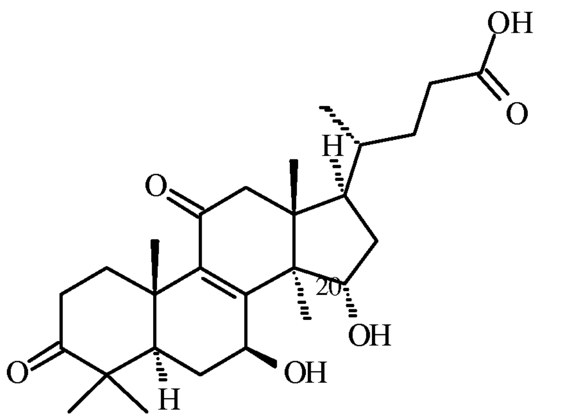

(I)

The present invention provides a pharmaceutical composition for the treatment or prophylaxis of a kidney disease. The pharmaceutical composition includes a therapeutically effective amount of the compound of formula (I); and at least another triterpenoid that is any of lucidenic acid C (LAC), lucidenic acid N (LAN), lucidenic acid $E_2$ ($LAE_2$), lucidenic acid A (LAA), lucidenic acid B (LAB), or lucidenic acid $D_2$ ($LAD_2$); and a pharmaceutically acceptable excipient.

According to the invention the pharmaceutical composition for use in the treatment or prophylaxis of a kidney

disease comprises the compound of formula (I), LAC, LAA, $LAD_2$, LAB, LAN, and $LAE_2$; and a pharmaceutically acceptable excipient; wherein the triterpenoid compound of formula (I) and LAC are present in about 5-15% by weight of the total triterpenoid in the pharmaceutical composition, LAB and LAN are respectively present in about 5-12% by weight of the total triterpenoid in the pharmaceutical composition, LAA and $LAD_2$ are respectively present in about 15-30% by weight of the total triterpenoid in the pharmaceutical composition, and $LAE_2$ is present in about 8-15% by weight of the total triterpenoid in the pharmaceutical composition.

The kidney disease treatable or preventable by the pharmaceutical composition of this disclosure is acute kidney injury (AKI) or chronic kidney disease (CKD). In one example, the kidney disease is AKI, particularly, acute kidney inflammation. In another example, the kidney disease is CKD, such as chronic kidney inflammation.

The combination of triterpenoids of this disclosure, specifically the combination of the compound of formula (I), LAC, LAA, $LAD_2$, LAB, LAN, and $LAE_2$, is present at a level of about 0.1 % to 99% by weight, based on the total weight of the pharmaceutical composition. In some embodiments, the combination of the triterpenoids of this disclosure is present at a level of at least 1% by weight, based on the total weight of the pharmaceutical composition. In certain embodiments, the combination of the triterpenoids of this disclosure is present at a level of at least 5% by weight, based on the total weight of the pharmaceutical composition. In still other embodiments, the combination of the triterpenoids of this disclosure is present at a level of at least 10% by weight, based on the total weight of the pharmaceutical composition. In still yet other embodiments, the combination of the triterpenoids is present at a level of at least 25% by weight, based on the total weight of the pharmaceutical composition.

The the compositions for use according to the present invention are used in a method which includes the step of administering to the subject a therapeutically effective amount of the composition defined above to improve or ameliorate the symptoms of the kidney disease.

According to an example, the r compositions are for use in a method which includes the step of administering the composition of the present invention to the subject to improve or ameliorate the symptoms of the kidney disease. The composition comprises the compound of formula (I), LAC, LAA, $LAD_2$, LAB, LAN, and $LAE_2$, and a pharmaceutically acceptable carrier, wherein the amount of the compound of formula (I), LAC, LAA, $LAD_2$, LAB, LAN, and $LAE_2$ are respectively about 10%, 10%, 10%, 8.5%, 15%, 25% and 25% by weight of the total triterpenoid in the pharmaceutical composition. Kidney diseases that may be treated or prevented from occurring include, but is not limited to, AKI and CKD. In one example, the kidney disease is AKI, particularly, acute kidney inflammation. In another example, the kidney disease is CKD, such as chronic kidney inflammation. The subject may be a mammal, preferably a human.

The details of one or more embodiments of this disclosure are set forth in the accompanying description below. Other features and advantages of the invention will be apparent from the detail descriptions, and from claims.

It is to be understood that both the foregoing general description and the following detailed description are by examples, and are intended to provide further explanation of the invention as claimed.

## BRIEF DESCRIPTION OF THE DRAWINGS

The accompanying drawings, which are incorporated in and constitute a part of the specification, illustrate various example systems, methods and other exemplified embodiments of various aspects of the invention. The present description will be better understood from the following detailed description read in light of the accompanying drawings, where,

**FIG 1A** illustrates the protective effect of the compound of formula (I) on cisplatin induced kidney cell apoptosis in accordance with one embodiment of the present disclosure;

**FIG 1B** illustrates the protective effect of the composition YKII-10 of example 1.2 on cisplatin induced kidney cell apoptosis in accordance with one embodiment of the present disclosure;

**FIG 2A** illustrates the inhibitory effect of the composition YKII-10 of example 1.2 on cisplatin induced increase in blood urea nitrogen in accordance with one embodiment of the present disclosure;

**FIG 2B** illustrates the inhibitory effect of the composition YKII-10 of example 1.2 on cisplatin induced increase in serum creatinine level in accordance with one embodiment of the present disclosure;

**FIG 3** illustrates the respective inhibitory effects of the compound of formula (I) and the composition YKII-10 of example 1.2 on lipopolysaccharide (LPS) induced increase in urea protein in accordance with one embodiment of the present disclosure;

**FIG 4A** illustrates the inhibitory effect of the composition YKII-10 of example 1.2 on aristolochic acid (AAI) induced increase in blood urea nitrogen in accordance with one embodiment of the present disclosure;

**FIG 4B** illustrates the inhibitory effect of the composition YKII-10 of example 1.2 on aristolochic acid (AAI) induced increase in serum creatinine level in accordance with one embodiment of the present disclosure;

**FIG 5** illustrates the effect of the composition YKII-10 of example 1.2 on glomerular filtration rate (GFR) in accordance with one embodiment of the present disclosure; and

**FIG 6** illustrates the effect of the composition YKII-10 of example 1.2 on relative estimated glomerular filtration rate (eGFR) in accordance with one embodiment of the present disclosure.

## DETAILED DESCRIPTION OF PREFERRED EMBODIMENTS

The detailed description provided below in connection with the appended drawings is intended as a description of the present disclosure and is not intended to represent the only forms in which the present disclosure may be constructed or utilized.

In the context of this disclosure, a number of terms shall be used.

The terms "treatment" and "treating" are used herein to include preventative (e.g., prophylactic), curative, or palliative treatment that results in a desired pharmaceutical and/or physiological effect. Preferably, the effect is therapeutic in terms of partially or completely curing or preventing the apotosis of kidney cells. Also, the term "treating" as used herein refers to application or administration of the compound or the composition of the present disclosure to a subject, who has a medical condition, a symptom of the condition, a disease or disorder secondary to the condition, or a predisposition toward the condition, with the purpose to partially or completely alleviate, ameliorate, relieve, delay onset of, inhibit progression of, reduce severity of, and/or reduce incidence of one or more symptoms or features of a particular disease, disorder, and/or condition. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition. As used herein, the symptom, disease, disorder or condition may be acute kidney injury (AKI) or chronic kidney disease (CKD). Treatment is generally "effective" if one or more symptoms or clinical markers are reduced as that term is defined herein.

The term "prophylaxis" as used herein means prevention against a future event. In the context of prophylaxis against kidney cells apoptosis that may potentially occur as a consequence of kidney cells being exposed under a toxic compound or a medicament, the prophylactic administration can occur before, contemporaneous with, and/or after the subject being exposed under the toxic compound or medicament. According to some embodiments of the present disclosure, the compound of formula (I) or a composition comprising the same is prophylactically administered to the subject before the subject is exposed to a toxic compound such as cisplatin, lipopolysaccharide (LPS) or aristolochic acid (AAI), that results in kidney inflammation and/or damage.

The terms "compounds," "compositions," "agent" and "medicament" are used interchangeably herein to refer to a compound or a composition of which, when administered to a subject such as a human or an animal induces a desired pharmacological and/or physiological effect by local and/or systemic action.

The terms "administered," "administering" and "administration" are used interchangeably herein to refer means either directly administering a compound or a composition of the present invention, or administering a prodrug, derivative or analog which will form an equivalent amount of the active compound of formula (I) within the body.

The term "subject" or "patient" refers to an animal including the human species that is treatable with the compositions and/or methods of the present invention. The term "subject" or "patient" intended to refer to both the male and female gender unless one gender is specifically indicated. Accordingly, the term "subject" or "patient" includes, but is not limited to, human, non-human primate such as any mammal, dog, cat, horse, sheep, pig, cow and etc., preferably a human, which may benefit from treatment by the compound of this disclosure. The terms "subject" and "patient" are used interchangeably in the present disclosure.

The term "an effective amount" as used herein refers to an amount effective, at dosages, and for periods of time necessary, to achieve the desired therapeutically result with respect to the treatment of a kidney disease. The effective amount of the compound of formula (I), its salt, ester or solvate may be ranged from about 0.1 mg/Kg/day to about 100 mg/Kg/day; preferably from about 1 mg/Kg/day to about 50 mg/Kg/day; more preferably from about 5 mg/Kg/day to about 10 mg/Kg/day. The effective amount may vary depends on the route of administration, and/or other the combinational use of other medicaments.

Throughout the specification, unless otherwise indicated to the contrary, the term "triterpenoid" herein means to encompass the triterpenoid, as well as its pharmaceutically acceptable salts, esters or solvates. For example, the triterpenoid compound of formula (I) of the present disclosure is meant to encompass the triterpenoid compound of formula (I), and its pharmaceutically acceptable salts, esters or solvates. The term "salt" refers herein as a salt which is formed by the interaction of a base including organic or inorganic types of bases, including bases formed from a metal of the IA or IIA group, ammonium and/or $N(alkyl)_4^+$ with an acid (such as the compound of formula (I) in this disclosure). The term "ester" herein refers to an ester of the compound of formula (I), in which the ester bond may be formed between the acid group of the compound of formula (I) and an alcohol, such as straight or branched $C_{1-6}$ alcohol; or between the hydroxyl group of the compound of formula (I) and an organic acid, such as R-COOH, wherein R is a straight or branched $C_{1-6}$ alkyl. The term "solvate" herein refers to a complex formed by the interaction of a compound (such as the compound of formula (I) in this disclosure) with surrounding solvent molecules, such as water, ethanol and etc.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1 %, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The present disclosure is based, at least in part, unexpected discovery that a triterpenoid isolated from the fruit bodies or mycelia of *Ganoderma lucidum* may suppress or inhibit apoptosis of kidney cells, and thereby results in a decrease in BUN and serum creatinine levels, and an increase in glomerular filtration rate (GFR). The results suggest that the triterpenoid of the present disclosure is useful as a therapeutic medicament for the treatment or prophylaxis of kidney disease, such as AKI and CKD.

Accordingly, there is provided triterpenoid compound of formula (I),

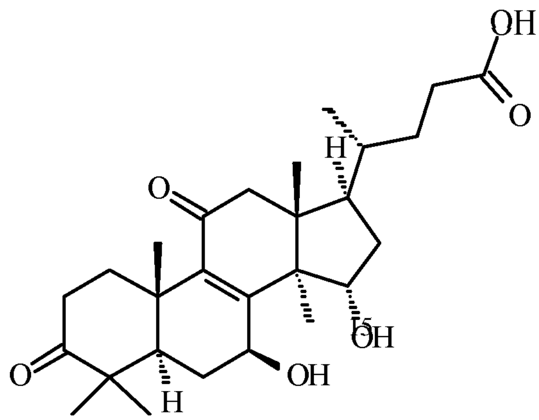

(I)

The compound of formula (I) is effective in suppressing cisplatin or aristolochic acid (AAI) induced kidney inflammation and/or damage manefesting in the increase in blood ura nitrogen and serum creatinine levels; as well as in ameloirating cisplatin or AAI induced kidney cell apoptosis; thus the compound of formula (I) may be used as a lead compound for the manufacture of a medicament for the treatment or prophylaxis of a kidney disease, such as AKI or CKD.

Inventors of the present disclosure provide as the present invention a combination of triterpenoids, in which each triterpenoid may be isolated from the fruit bodies or mycelia of *Ganoderma lucidum,* this composition being as defined above, for use in the treatment or prophylaxis of a kidney disease. This novel combination of triterpenoids is also effective in suppressing cisplatin induced kidney cell apoptosis, ameliorating LPS or AAI induced kidney inflammation and/or damage; thus may also be used for the manufacture of a medicament for the treatment or prophylaxis of a kidney disease, such as AKI or CKD.

It is therefore the objective of this disclosure to provide a pharmaceutical composition, which comprises the novel combination of triterpenoids described above, for the treatment or prophylaxis of a kidney disease. The pharmaceutical composition includes a therapeutically effective amount of the compound of formula (I), LAC, LAN, $LAE_2$, LAA, LAB, and $LAD_2$; and a pharmaceutically acceptable carrier.

According to the invention, the amounts of the compound of formula (I) and LAC are respectively about 5-15% by weight of the total triterpenoid in the pharmaceutical composition, such as 5, 5.5, 6, 6.5, 7, 7.5, 8, 8.5, 9, 9.5, 10, 10.5, 11, 11.5, 12, 12.5, 13, 13.5, 14, 14.5 and 15% by weight of the total triterpenoid in the pharmaceutical composition. In a preferred embodiment, the amounts of the compound of formula (I) and LAC are respectively about 10% by weight of the total triterpenoid in the pharmaceutical composition.

According to the invention, the amounts of LAA and $LAD_2$ are respectively about 15-30% by weight of the total triterpenoid in the pharmaceutical composition, such as 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 and 30% by weight of the total triterpenoid in the pharmaceutical composition. In a preferred embodiment, the amount of LAA and $LAD_2$ are respectively about

25% by weight of the total triterpenoid in the pharmaceutical composition.

According to the invention, the amount of $LAE_2$ is about 8-15% by weight of the total triterpenoid in the pharmaceutical composition, such as 8, 8.5, 9, 9.5, 10, 10.5, 11, 11.5, 12, 12.5, 13, 13.5, 14, 14.5 and 15% by weight of the total triterpenoid in the pharmaceutical composition. In a preferred embodiment, the amount of $LAE_2$ is about 12% by weight of the total triterpenoid in the pharmaceutical composition.

According to the invention, the amounts of LAN and LAB are respectively about 5-12% by weight of the total triterpenoid in the pharmaceutical composition, such as 5, 5.5, 6, 6.5, 7, 7.5, 8, 8.5, 9, 9.5, 10, 10.5, 11, 11.5, and 12% by weight of the total triterpenoid in the pharmaceutical composition. In a preferred embodiment, the amounts of LAN and LAB are respectively about 8.5% by weight of the total triterpenoid in the pharmaceutical composition.

In one preferred embodiment, the pharmaceutical composition comprises the compound of formula (I), LAC, LAN, $LAE_2$, LAA, LAB, and $LAD_2$; and a pharmaceutically acceptable carrier; and wherein the amounts of the compound of formula (I), LAC, LAN, $LAE_2$, LAA, and $LAD_2$ are respectively about 10%, 10%, 8.5%, 15%, 25% and 25% by weight of the total triterpenoid in the pharmaceutical composition.

The respective triterpenoids in the pharmaceutical composition of the present disclosure may be purified from the fruit bodies or mycelia of *Ganoderma lucidum* by methods well known in the art or by similar methods set forth in example 1 of the present disclosure. Whether the raw material used for isolating triterpenoids is the fruit bodies or the mycelia, such method in general involves extracting the plant with a solvent, preferably an alcoholic solution, at a temperature above room temperature; followed by subjecting the extract with column chromatography, which includes but is not limited to, high performance liquid chromatography (HPLC), reverse phase liquid chromatography and etc.; and concentrating and drying, until a dried powder is obtained.

Generally, the triterpenoids of the present disclosure, specifically the combination of the compound of formula (I), LAC, LAN, $LAE_2$, LAA, LAB, and $LAD_2$, is present at a level of about 0.1 % to 99% by weight, based on the total weight of the pharmaceutical composition. In some embodiments, the combination of the triterpenoids of the present disclosure is present at a level of at least 1% by weight, based on the total weight of the pharmaceutical composition. In certain embodiments, the combination of the triterpenoids of the present disclosure is present at a level of at least 5% by weight, based on the total weight of the pharmaceutical composition. In still other embodiments, the combination of the triterpenoids of the present disclosure is present at a level of at least 10% by weight, based on the total weight of the pharmaceutical composition. In still yet other embodiments, the combination of the triterpenoids is present at a level of at least 25% by weight, based on the total weight of the pharmaceutical composition.

Kidney disease that may be treated by the compound of formula (I) or by the pharmaceutical composition of this disclosure include, but are not limited to, acute kidney injury and chronic kidney disease. In one example, the compound of formula (I) is used to treat AKI, such as cisplatin or AAI-induced kidney inflammation. In another example, the pharmaceutical composition of this disclosure, or the pharmaceutical composition comprising the compound of formula (I), is employed to treat AKI.

The medicament or said pharmaceutical composition is prepared in accordance with acceptable pharmaceutical procedures, such as described in Remington's Pharmaceutical Sciences, 17th edition, ed. Alfonoso R. Gennaro, Mack Publishing Company, Easton, Pa (1985). Pharmaceutically acceptable excipients are those that are compatible with other ingredients in the formulation and biologically acceptable.

The pharmaceutical composition of the present disclosure may be administered by any suitable route, for example, orally in capsules, suspensions or tablets or by parenteral administration. Parenteral administration can include, for example, systemic administration such as intramuscular, intravenous, subcutaneous, or intraperitoneal injection. The pharmaceutical composition can also be administered transdermally either topically or by inhalation (e.g., intrabronichial, intranasal, oral inhalation or intranasal drops), or rectally, alone or in combination with conventional pharmaceutically acceptable excipients. In preferred embodiments, the pharmaceutical composition of the present disclosure are administered orally (e.g., dietary) to the subject.

For oral administration, the pharmaceutical composition of the present disclosure may be formulated into tablets containing various excipients such as microcrystalline cellulose, sodium citrate, calcium carbonate, dicalcium phosphate, and glycine; along with various disintegrants such as starch, alginic acid and certain silicates; together with granulation binders like polyvinylpyrrolidone, sucrose, gelatin and acacia. Additionally, lubricating agents such as magnesium stearate, sodium lauryl sulfate and talc may be added. Solid composition may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and if so desired, emulsifying and/or suspending agents as well, together with diluents such as water, ethanol, propylene glycol, glycerin and a combination thereof.

For parenteral administration, the novel combination of triterpenoids of the present disclosure may be formulated into liquid pharmaceutical compositions, which are sterile solutions, or suspensions that can be administered by, for example, intravenous, intramuscular, subcutaneous, or intraperitoneal injection. Suitable diluents or solvent for manufacturing sterile injectable solution or suspension include, but are not limited to, 1,3-butanediol, mannitol, water, Ringer's solution, and isotonic sodium chloride solution. Fatty acids, such as oleic acid and its glyceride derivatives are also useful for preparing injectables, as are natural pharmaceutically-acceptable oils, such as olive oil or castor oil. These oil solutions or suspensions may also contain alcohol diluent or carboxymethyl cellulose or similar dispersing agents. Other commonly used surfactants such as Tweens or Spans or other similar emulsifying agents or bioavailability enhancers that are commonly used in manufacturing pharmaceutically acceptable dosage forms can also be used for the purpose of formulation.

For transmucosal administration, the medicament or said pharmaceutical compositions of the present disclosure may also be formulated in a variety of dosage forms for mucosal application, such as buccal and/or sublingual drug dosage units for drug delivery through oral mucosal membranes. A wide variety of biodegradable polymeric excipients may be used that are pharmaceutically acceptable, provide both a suitable degree of adhesion and the desired drug release profile, and are compatible with the active agents to be administered and any other components that may be present in the buccal and/or sublingual drug dosage units. Generally, the polymeric excipient comprises hydrophilic polymers that adhere to the wet surface of the oral mucosa. Examples of polymeric excipients include, but are not limited to, acrylic acid polymers and copolymers; hydrolyzed polyvinylalcohol; polyethylene oxides; polyacrylates; vinyl polymers and copolymers; polyvinylpyrrolidone; dextran; guar gum; pectins; starches; and cellulosic polymers.

It will be appreciated that the dosage of the pharmaceutical composition of the present disclosure will vary from patient to patient not only for the particular route of administration, and the ability of the composition to elicit a desired response in the patient, but also factors such as disease state or severity of the condition to be alleviated, age, sex, weight of the patient, the state of being of the patient, and the severity of the pathological condition being treated, concurrent medication or special diets then being followed by the patient, and other factors which those skilled in the art will recognize, with the appropriate dosage ultimately being at the discretion of the attendant physician. Dosage regimens may be adjusted to provide the improved therapeutic response. A therapeutically effective amount is also one in which any toxic or detrimental effects of the composition are outweighed by the therapeutically beneficial effects. Preferably, the compositions of the present disclosure are administered at a dosage and for a time such that the number and/or severity of the symptoms are decreased.

The pharmaceutical composition of the present disclosure may be administered to a subject in a dose from about 0.1 mg to about 100 mg per kilogram of body weight per day, such as 0.1, 0.5, 1, 2, 5, 7, 9, 10, 12, 15, 17, 19, 20, 22, 25, 27, 29, 30, 32, 35, 37, 39, 40, 42, 45, 47, 49, 50, 52, 55, 57, 59, 60, 62, 65, 67, 69, 70, 72, 75, 77, 79, 80, 82, 85, 87, 89, 90, 92, 95, 97, 99 and 100 mg/Kg/day; preferably from about 1 to 50 mg per kilogram of body weight per day, such as 1, 2, 5, 7, 9, 10, 12, 15, 17, 19, 20, 22, 25, 27, 29, 30, 32, 35, 37, 39, 40, 42, 45, 47, 49, and 50 mg/Kg/day; more preferably from about 5 to 10 mg per kilogram of body weight per day, such as 5, 7, 8, 9, and 10 mg/Kg/day. The dose may be administered once a day or be at least two, three, four, or five times a day.

The present disclosure also provides the compositions according to the present invention for use in the treatment or prophylaxis of a kidney disease, wherein this achieved by a method which includes the step of administering to the subject an effective amount of the compound of formula (I) or the composition of the present disclosure to the subject to improve or ameliorate the symptoms of the kidney disease, such as protecting the kidney cells from apoptosis or improving glomerular filtration rate (GFR).

According to another example, the method includes the step of administering to the subject a therapeutically amount of the composition of the present disclosure to improve GFR of the subject for at least 4 folds, as compared to the GFR of the subject before treatment. Preferably, the composition comprises the compound of formula (I), LAC, LAA, $LAD_2$, LAB, LAN, and $LAE_2$, and a pharmaceutically acceptable carrier, wherein the amount of the compound of formula (I), LAC, LAA, $LAD_2$, LAB, LAN, and $LAE_2$ are respectively about 10%, 10%, 10%, 8.5%, 15%, 25% and 25% by weight of the total triterpenoid in the pharmaceutical composition. The composition is administered to a mammal, preferably human, by any route that may effectively transports the active ingredient(s) of the composition to the appropriate or desired site of action, such as oral, nasal, pulmonary, transdermal, such as passive or iontophoretic delivery, or parenteral, e.g., rectal, depot, subcutaneous, intravenous, intramuscular, intranasal, ophthalmic solution or an ointment.

The present invention will now be described more specifically with reference to the following embodiments, which are provided for the purpose of demonstration rather than limitation.

## EXAMPLES

### Materials and Methods

*Cell Culture*

Madin-Darby canine kidney (MDCK) cells (1.5 x $10^5$ cells/mL) were cultured and maintained in Dulbecco's modified Eagle medium (DMEM) supplemented with 10% fetal bovine serum (FBS), 100 units/mL penicillin, 100 ng/mL

streptomycin, 2mM L-glutamine, non-essential amino acids and sodium pyruvate, and maintained in 5% $CO_2$ at 37 °C until reached 80% confluence, then were subject to cell passages.

*Animals*

Blb/c mice were kept in pathogen-free environment under 12:12 light-dark cycle with food (laboratory rodent diet 5001 purchased from PMI Nutrition International Inc. MO, USA) and water (i.e., distilled water) provided *ad libitum,* ambient temperature and relative humidity were respectively set at 22±3 °C and 50±20%. All procedures involving animal studies of the present disclosure comply with the "Guideline for the Care and Use of Laboratory Animals" issued by The Chinese-Taipei Society of Laboratory Animal Sciences.

**EXAMPLE 1 Isolation of the compound of formula (I) and the preparation of the composition of the present disclosure**

**1.1 Identification and characterization of the compound of formula (I)**

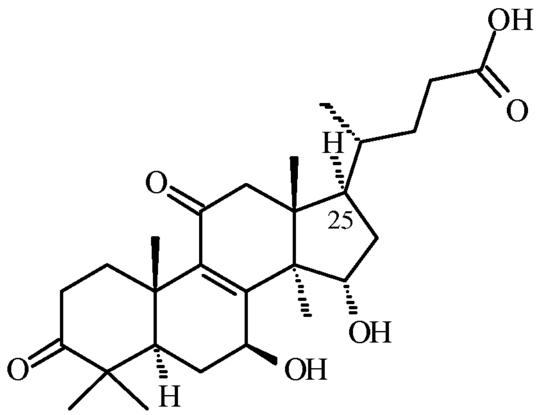

(I)

The fruiting bodies of *Granoderma lucidum* (50 g) were grounded to powders, and extracted with ethanol in a ratio of *G. lucidum* (weight) to ethanol (volume) = 1:24 at 37 °C for a day, the extract was then subject to concentration. The extraction and concentration steps were repeated several times, and the respective extracts were filterted, combined and dried with a yield crude extract of about 10% (i.e., 5 g of crude extract). The resultant crude extract of *G. lucidum* was then vacuumed dried at about 40 °C to remove any residual solvent.

5 g of the crude extract of *G. lucidum* prepared above was subsequently subject to extraction with ethyl acetate (EA), the EA extract was then concentrated, and vacuumed dried at about 40 °C to remove residual solvent. The yield of the EA extract of *G. lucidum* was about 40% (or 2.0 g).

The EA extract of *G. lucidum* was dissolved in ethanol and subjected to semi-preparative high pressure liquid chromatography, in which the column was eluted with cyanomethane and 2% acetic acid, and the compound of formula (I) was identified therein with a yield of about 0.4% (8 mg).

The $^{13}C$ and $^{1}H$ NMR spectra, as well as heteronuclear multiple bond correlation (HMBC) spectra of the compound of formula (I) are summarized in Table 1.

Table 1 The spectra data of the compound of formula (I)

| C No. | $\delta_C$ | $\delta_H$ | HMBC (H to C) |
|---|---|---|---|
| 1 | 35.5 | 1.46 ($\alpha$, $\mu$), 2.85 ($\beta$, ddd, 6.6, 7.0, 13.6) | 2, 3, 5, 10, 19 |
| 2 | 34.3 | 2.49 (2H, dd, 7.0, 8.4). | 1, 3, 4, 10 |
| 3 | 217.0 | | |
| 4 | 46.8 | | |
| 5 | 48.8 | 1.68 (m) | 1, 4, 10, 19, 26, 27 |

(continued)

| C No. | $\delta_C$ | $\delta_H$ | HMBC (H to C) |
|---|---|---|---|
| 6 | 28.9 | 2.04 ($\alpha$, dd, 3.6, 11.3), 1.66 ($\beta$) | 4, 8, 10 |
| 7 | 68.9 | 4.63 (dd, 6.9, 9.5) | 8, 9, 25 |
| 8 | 159.1 | | |
| 9 | 140.4 | | |
| 10 | 38.0 | | |
| 11 | 199.8 | | |
| 12 | 51.8 | 2.75 ($\alpha$, d, 15.9), 2.52, ($\beta$, d, 15.9) | 9, 11, 13, 14, 18 |
| 13 | 46.7 | | |
| 14 | 53.9 | | |
| 15 | 72.6 | 4.80 (dd, 6.9, 9.6) | 8, 14, 25 |
| 16 | 36.4 | 1.84 ($\alpha$, m), 1.88 ($\beta$, m) | 25 |
| 17 | 48.5 | 1.78 (m) | 21 |
| 18 | 17.2 | 0.96 (s) | 12, 13, 14, 17 |
| 19 | 19.4 | 1.29 (s) | 1, 5, 9, 10, 27 |
| 20 | 35.7 | 1.47 (m) | 21 |
| 21 | 18.1 | 0.89 (d, 6.5) | 17, 20, 22 |
| 22 | 30.9 | 1.81 (m) | 21 |
| 23 | 30.9 | 2.41 (m), 2.31 (ddd, 8.0, 8.1, 16.0) | 21 |
| 24 | 177.9 | | |
| 25 | 19.4 | 1.26 (s) | 8, 13, 14, 15 |
| 26 | 27.3 | 1.12 (s) | 3, 4, 5, 27 |
| 27 | 20.7 | 1.11 (s) | 3, 4, 5, 26 |

The molecular of formila (I) was determined as $C_{27}H_{40}O_6$ from its HRMS *m*/*z*=460.2834 as well as from the $^{13}C$ and $^1H$ NMR.

The MS of formula (I) showed fragmented ions at *m*/*z* (rel. int.): 460 [M+] (53.56), 442 (22.56), 339 (16.46), 322 (100), 304 (46.41); and its IR spectra showed absorption bands at 3445, 1736, 1707, 1661 $cm^{-1}$ ; and UV spectra showed maximum absorption (MeOH, $\lambda_{max}$) at 252 nm; with a melting point between 124 - 126 °C.

**1.2 Preparation of the pharmaceutical composition YKII-10 comprising the compound of formula (I)**

The pharmaceutical composition of the present disclosure (or YKII-10 composition) for treating kidney disease is prepared by mixing the compound of formula (I) of example 1.1 with other triterpenoids in accordance with the formulation listed in Table 2. The triterpenoids of Table 2 may be obtained by extracting the fruting bodies and/or mycelia of *Grandoderma spp.* with any solvent(s), followed by separation with suitable chromatography.

Table 2 The YKII-10 formulation

| Name of Compound | % of Total Triterpenoids |
|---|---|
| LAA | 24.2 % |
| LAB | 8.3 % |
| LAC | 10.7 % |
| $LAD_2$ | 25.9 % |

(continued)

| Name of Compound | % of Total Triterpenoids |
|---|---|
| $LAE_2$ | 12.3 % |
| LAN | 8.6 % |
| The compound of formula (I) | 10 % |
| Total Triterpenoids | 100 % |

**Example 2 Both the compound of formula (I) and the YKII-10 composition of example 1.2 are effective in ameliorating or improving cisplatin induced kidney inflammation**

In the present example, *in vitro* kidney cell apoptosis and *in vivo* kidney inflammation were respectively used as indicators for assessing the protective effect rendered by the compound of formula (I) and the YKII-10 composition of example 1.2.

**2.1 Cisplatin induced *In Vitro* kidney cell apoptosis**

MDCK cells were cultured in according to procedures described in the "Materials and Methods" section. To start the experiment, MDCK cells were first incubated with 50 μM cisplatin for 4 hrs, then various concentrations of the compound of formula (I) (50μg/mL or 100 μg/mL) or the YKII-10 composition of example 1.2 (25 to 100 μg/mL) were given to cisplatin-treated cells and further incubated for another 24 hrs. Mitochondria membrane potentials were then measured by staining the live cells with fluorescent dyes (e.g., 50 nM DiOC6), in which 50 nM DiOC6 were added to cells and incubated at 37 °C for 30 min. The cells were then washed twice with phosphate buffer solution before subjecting to microscopy analysis using the inverted fluorescent microscope. Since only live cells are capable of taking up the DiOC6 dye, hence the test is useful for evaluating any effect of a candidate compound (e.g., the compound of formula (I) or the YKII-10 composition of example 1.2) on the progression of apoptosis. Results are illustrated in FIGs 1 A and 1 B.

As the bar graph of FIG 1 A illustrated, the compound of formula (I) (50μg/mL or 100 μg/mL) is capable of reversing cisplatin-induced apoptosis of MDCK cell, with about 80% recovery on the membrane potential at the dose of 100 μg/mL. Similar results were also observed when the YKII-10 composition of example 1.2 was employed, in whcih the membrane potentail of MDCK cells increased in a dose dependent manner from 25 to 100 μg/mL. Taken together, the results from this example indicate that both the compound of formula (I) and the YKII-10 composition comprising the compound of formula (I) are both effective in reversing cisplatin induced apoptosis, thus the compound of formula (I) and the composition of example 1.2 may be used to treat or ameoliorate kidney disease (e.g., kidney inflammation).

**2.2 Cisplatin induced *In Vivo* kidney inflammation**

On the first day of this experiment, Blb/c mice (6 weeks old) were randomly assigned to two groups, with the mice in the test group being orally given the YKII-10 composition of example 1.2 (10 mL/Kg) for 5 days, and the mice in the control group were given just the distilled water. All animals were injected intraperitonealy with cisplatin (15 mg/Kg) on the second day, and blood samples were collected on the fifth day, then analyzed for blood urine nitrogen (BUN), and creatinine (CRE), which were used as indicators for assessing the kidney function. Results are illustrated in FIG 2.

As depicted in FIGs 2A and FIG 2B, both the levels of BUN and CRE increased after the injection of cisplatin, and this cisplatin induced increases in BUN and CRE were both suppressed in the presence of the YKII-10 composition of example 1.2, in which the compound of formula (I) is included. Thus, the data supports the proposition that the composition comprising the compound of formula (I) of the present invention may be used for the treatment or prophylaxis of kidney disease.

**Example 3 Both the compound of formula (I) and the YKII-10 composition of example 1.2 are effective in ameliorating or improving lipopolysaccharide induced kidney inflammation**

In this example, to evaluate the therapeutic or prophylactic function of the compositon of example 1.2, animals were first injected intraperitoneally with lipopolysaccharide (LPS) to induce *in vivo* kidney inflammation before being subect to the treatment of the compositon of example 1.2.

Blb/c mice (6 weeks old) were randomly assigned to two groups, and injected intraperitoneally with lipopolysaccharide (LPS, 2.5 mg/Kg/day) for 5 consecutive days so as to induce kidney inflammation. In the mean time, mice in the test group were orally fed with either the compound of formula (I) (at a dosage of 100 mg/Kg) or the YKII-10

composition of example 1.2 (at a dosage of 250 mg/Kg), while the animals in the control group were given just the water. Unine sampls were respectively collected on the fifth and the tenth day after the experiment started, and analyzed for the level of urine protein therein. The results are depicted in FIG 3.

As depicted in FIG 3, both the compound of formula (I) and the YKII-10 composition (i.e., the composition of example 1.2) are effective in reducing the urine protein level in the test animals, which support the hypothesis that the the compound of formula (I) or the composition comprising the compound of formula (I) may be used for the treatment or prophylaxis of kidney disease.

**Example 4 YKII-10 composition of example 1.2 is effective in ameliorating or improving *in vivo* aristolochic acid (AAI) induced acute kidney injury (AKI)**

**4.1 AAI induced *In vivo* acute kidney injury**

Blb/c mice (6 weeks old) were randomly assigned to two groups, and orally fed with AAI (2.5 mg/Kg/day) for 5 consecutive days, so as to induce AKI. Mice in the test group were orally fed with the YKII-10 composition of example 1.2 (at a dosage of 300 mg/Kg) for 12 consecutive days, while the animals in the control group were given just the water. Blood samples were respectively collected on the 12th day after the experiment started, and analyzed for the level of BUN and CRE. The results are depicted in FIGs 4A and 4B.

As depicted in FIGs 4A and 4B, the YKII-10 composition (i.e., the composition of example 1.2) is effective in reducing both the AAI-induced increase in BUN and CRE in the test animals, which support the hypothesis that the composition comprising the compound of formula (I) may be used for the treatment or prophylaxis of kidney disease, including AKI.

**4.2 AAI induced failure of Glomerular filtration rate (GFR)**

GFR is the most relevant metric for determining the extent of AKI and progression of chronic kidney disease (CKD). Reduction of GFR secondary to kidney injury, either acute or chronic, are accompanied by increases in blood urine nitrogen (BUN) and serum creatinine (CRE) level. In this example, GFR was measured by monitoring the dissipiation of the fluorescence signal of a sugar, i.e., inulin labeled with a fluorescent marker, which can only be metabolized through glomercular filtration.

Blb/c mice (6 weeks old) were randomly assigned to two groups, in which mice in the test group were orally fed with the YKII-10 composition (300 mg/Kg/day) for 8 days, and AAI (2.5 mg/Kg/day) was injected intraperitoneally on the fourth day, and continued the injection for 5 days. By contrast, mice in the control group received only water as their treatment. On the eighth day of the experiment, fluorescent inulin (GFR-Vivo 680, $2 \times 10^{-8}$ mol) was injected into the test animals, and the animals were monitored with a Fluorescence Tomography System (FMT 4000) with images taken at 1, 5, 15, 30 and 45 minutes, respectively. Respective GFR at various time points are calculated and the results are depicted in FIG 5.

As depicted in FIG 5, AAI treatment knocked down the GFR from its normal value of about 250 $\mu$L/min to about 25 $\mu$L/min, about 10 folds decreases; however, if the mice were pre-treated with YKII-10 composition of this disclosure, it may have a protective effect on GFR, though GFR value still did not return to its normal value, yet damage to GFR was less significant (about 100$\mu$L/min).

**Example 5 YKII-10 composition of example 1.2 is effective in ameliorating or improving *in vivo* kidney function**

In this example, animals were subjected to surgery to remove part of their kidneys and thereby resulted in a drop in their respective kidney funcitons to a level that was about 1/6 of a normal value, so as to mimic the kidney function of a subject suffering from chronic kidney disease (CKD). The animals were then treated with the YKII-10 composition of example 1.2 and its effect on enhancing the kidney function was evulated.

Adult male rats were randomly assigned to two groups (3 rats/group), and 2/3 of the left hand side kidney of each rat in both groups was surgically removed. The animals were left to recover for about a week, then their right hand side kindeys were completedly removed by sugery. Such procedure would render the kidney function of the test animal dropped to a level that is about 1/6 of that of a normal animal, thus may mimic the kidney function of a CKD subject. Two weeks after the surgery, animals in the test groups were orally fed with 300 mg/Kg of the YKII-10 composition of example 1.2 on a daily basis, whereas the control animals were merely given water as their treatments. Blod samples were collected every two weeks from the tail of each animals, and estimated GFR (eGFR) of each sample was then claculated by use of the following equation based on blood creatinine level obtained from each sample, and results are illustrated in FIG 6.

$$eGFR\ (mL/min/1.73\ m^2) = 186 \times (Creatinine\ level)^{-1.154} \times (30)^{-0.203}$$

As depicted in **FIG 6**, eGFR of each ratl in the test group is about 44% of that of a normal rat. 6 weeks after the surgery, the kidney function of each rats in the test group has slowly gone up to about 59% of that of a normal rat, indicating about 15% increases in the kidney function; whereas the kidney function of each rat in the control group stayed relatively the same, that is, without any sign of improving. Student T-test analysis indicated that the 15% increase in kidney funciton as illustrated in **FIG 6** is statistically significant, as compared with that of the control.

Taken together, results in this example validate the findings in other examples, and support the hypothesis that the composition comprising the compound of formula (I), may be used for the treatment or prophylaxis of kidney disease, including AKI and CKD.

## Claims

1. A pharmaceutical composition for the treatment or prophylaxis of a kidney disease comprising a triterpenoid compound of formula (I),

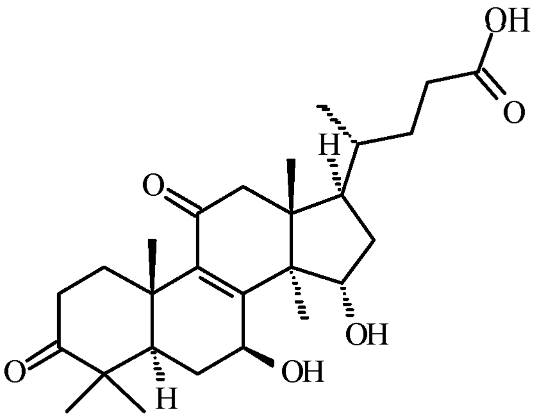

(I)

lucidenic acid C (LAC), lucidenic acid B (LAB), lucidenic acid N (LAN), lucidenic acid A (LAA), lucidenic acid $D_2$ ($LAD_2$), lucidenic acid $E_2$ ($LAE_2$); and a pharmaceutically acceptable excipient;
wherein:

the triterpenoid compound of formula (I) and LAC are respectively present in about 5-15% by weight of the total triterpenoid in the pharmaceutical composition, LAB and LAN are respectively present in about 5-12% by weight of the total triterpenoid in the pharmaceutical composition, LAA and $LAD_2$ are respectively present in about 15-30% by weight of the total triterpenoid in the pharmaceutical composition, and $LAE_2$ is present in about 8-15% by weight of the total triterpenoid in the pharmaceutical composition.

2. The pharmaceutical composition for use according to claim 1, wherein the triterpenoid compound of formula (I) and LAC are respectively present in about 10% by weight of the total triterpenoid in the pharmaceutical composition.

3. The pharmaceutical composition for use according to claim 1, wherein the LAB and LAN are respectively present in about 8.5% by weight of the total triterpenoid in the pharmaceutical composition.

4. The pharmaceutical composition for use according to claim 1, wherein the LAA and $LAD_2$ are respectively present in about 25% by weight of the total triterpenoid in the pharmaceutical composition.

5. The pharmaceutical composition for use according to claim 1, wherein the $LAE_2$ is present in about 12% by weight of the total triterpenoid in the pharmaceutical composition.

6. The pharmaceutical composition for use according to claim 1, wherein the subject is a human.

7. The pharmaceutical composition for use according to claim 1, wherein the kidney disease is acute kidney injury or chronic kidney disease.

## Patentansprüche

1. Pharmazeutische Verbindung für die Behandlung oder Prophylaxe einer Nierenerkrankung umfassend eine Triterpenoid - Verbindung der Formel (I),

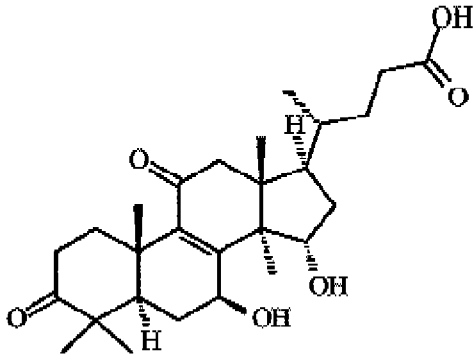

(I)

Lucidensäure C (LAC), Lucidensäure B (LAB), Lucidensäure N (LAN), Lucidensäure A (LAA), Lucidensäure $D_2$ ($LAD_2$), Lucidensäure $E_2$ ($LAE_2$) und einen pharmazeutisch zulässigen Hilfsstoff,
wobei
die Triterpenoid - Verbindung der Formel (I) und LAC jeweils mit ungefähr 5-15 Gewichts-% des gesamten Triterpenoids in der pharmazeutischen Verbindung vorliegen, wobei LAB und LAN jeweils mit ungefähr 5-12 Gewichts-% des gesamten Triterpenoids in der pharmazeutischen Verbindung vorliegen, wobei LAA und $LAD_2$ jeweils mit ungefähr 15-30 Gewichts-% des gesamten Triterpenoids in der pharmazeutischen Verbindung vorliegen und wobei $LAE_2$ mit ungefähr 8-15 Gewichts-% des gesamten Triterpenoids in der pharmazeutischen Verbindung vorliegt.

2. Pharmazeutische Verbindung für eine Verwendung nach Anspruch 1, wobei die Triterpenoid - Verbindung der Formel (I) und LAC jeweils mit 10 Gewichts-% des gesamten Triterpenoids in der pharmazeutischen Verbindung vorliegen.

3. Pharmazeutische Verbindung für eine Verwendung nach Anspruch 1, wobei LAB und LAN jeweils mit ungefähr 8,5 Gewichts-% des gesamten Triterpenoids in der pharmazeutischen Verbindung vorliegen.

4. Pharmazeutische Verbindung für eine Verwendung nach Anspruch 1, wobei LAA und $LAD_2$ jeweils mit ungefähr 25 Gewichts-% des gesamten Triterpenoids in der pharmazeutischen Verbindung vorliegen.

5. Pharmazeutische Verbindung für eine Verwendung nach Anspruch 1, wobei $LAE_2$ mit ungefähr 12 Gewichts-% des gesamten Triterpenoids in der pharmazeutischen Verbindung vorliegt.

6. Pharmazeutische Verbindung für eine Verwendung nach Anspruch 1, wobei das Subjekt ein Mensch ist.

7. Pharmazeutische Verbindung für eine Verwendung nach Anspruch 1, wobei die Nierenerkrankung eine akute Nierenverletzung oder eine chronische Nierenerkrankung ist.

## Revendications

1. Composition pharmaceutique pour le traitement ou la prophylaxie d’une maladie rénale comprenant un composé à base de triterpénoïde de la formule (I),

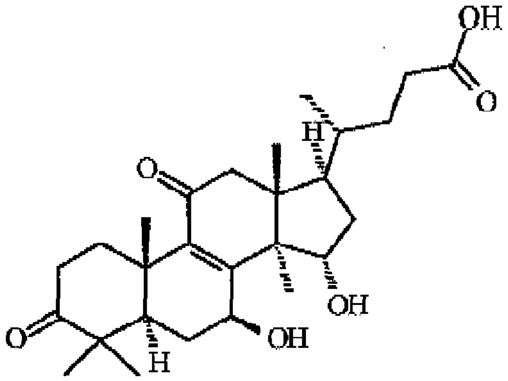

(I)

de l'acide lucidénique C (LAC), de l'acide lucidénique B (LAB), de l'acide lucidénique N (LAN), de l'acide lucidénique A (LAA), de l'acide lucidénique $D_2$ ($LAD_2$),de l'acide lucidénique $E_2$ ($LAE_2$) et un excipient pharmaceutiquement acceptable,

cependant que le composé à base de triterpénoïde de la formule (I) et le LAC sont présents respectivement dans environ 5 à 15% en poids du triterpénoïde total de la composition pharmaceutique, le LAB et le LAN sont présents respectivement dans environ 5 à 12% en poids du triterpénoïde total de la composition pharmaceutique, le LAA et le $LAD_2$ sont présents respectivement dans environ 15 à 30% en poids du triterpénoïde total de la composition pharmaceutique et le $LAE_2$ est présent dans environ 8 à 15% en poids du triterpénoïde total de la composition pharmaceutique.

2. Composition pharmaceutique pour utilisation selon la revendication 1, le composé à base de triterpénoïde de la formule (I) et le LAC étant présents respectivement dans environ 10% en poids du triterpénoïde total de la composition pharmaceutique.

3. Composition pharmaceutique pour utilisation selon la revendication 1, le LAB et le LAN étant présents respectivement dans environ 8,5% en poids du triterpénoïde total de la composition pharmaceutique.

4. Composition pharmaceutique pour utilisation selon la revendication 1, le LAA et le $LAD_2$ étant présents respectivement dans environ 25% en poids du triterpénoïde total de la composition pharmaceutique.

5. Composition pharmaceutique pour utilisation selon la revendication 1, le $LAE_2$ étant présent dans environ 12% en poids du triterpénoïde total de la composition pharmaceutique.

6. Composition pharmaceutique pour utilisation selon la revendication 1, le sujet étant un être humain.

7. Composition pharmaceutique pour utilisation selon la revendication 1, la maladie rénale étant l'insuffisance rénale aiguë ou l'insuffisance rénale chronique.

(A)

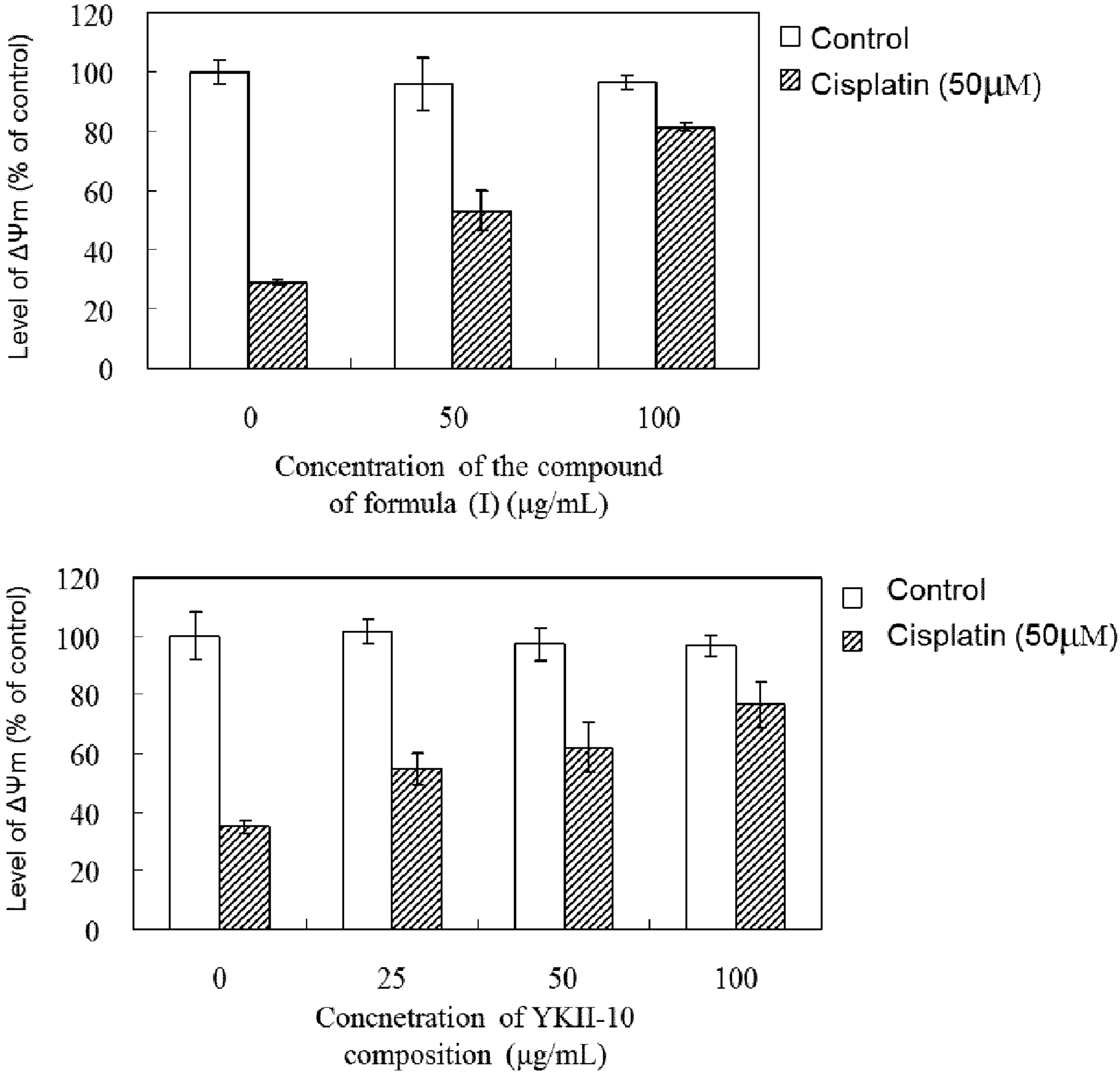
Level of ΔΨm (% of control)
120
100
80
60
40
20
0
Control
Cisplatin (50μM)
0
50
100
Concentration of the compound
of formula (I) (μg/mL)
(B)
Level of ΔΨm (% of control)
120
100
80
60
40
20
0
Control
Cisplatin (50μM)
0
25
50
100
Concnetration of YKII-10
composition (μg/mL)

FIG 1

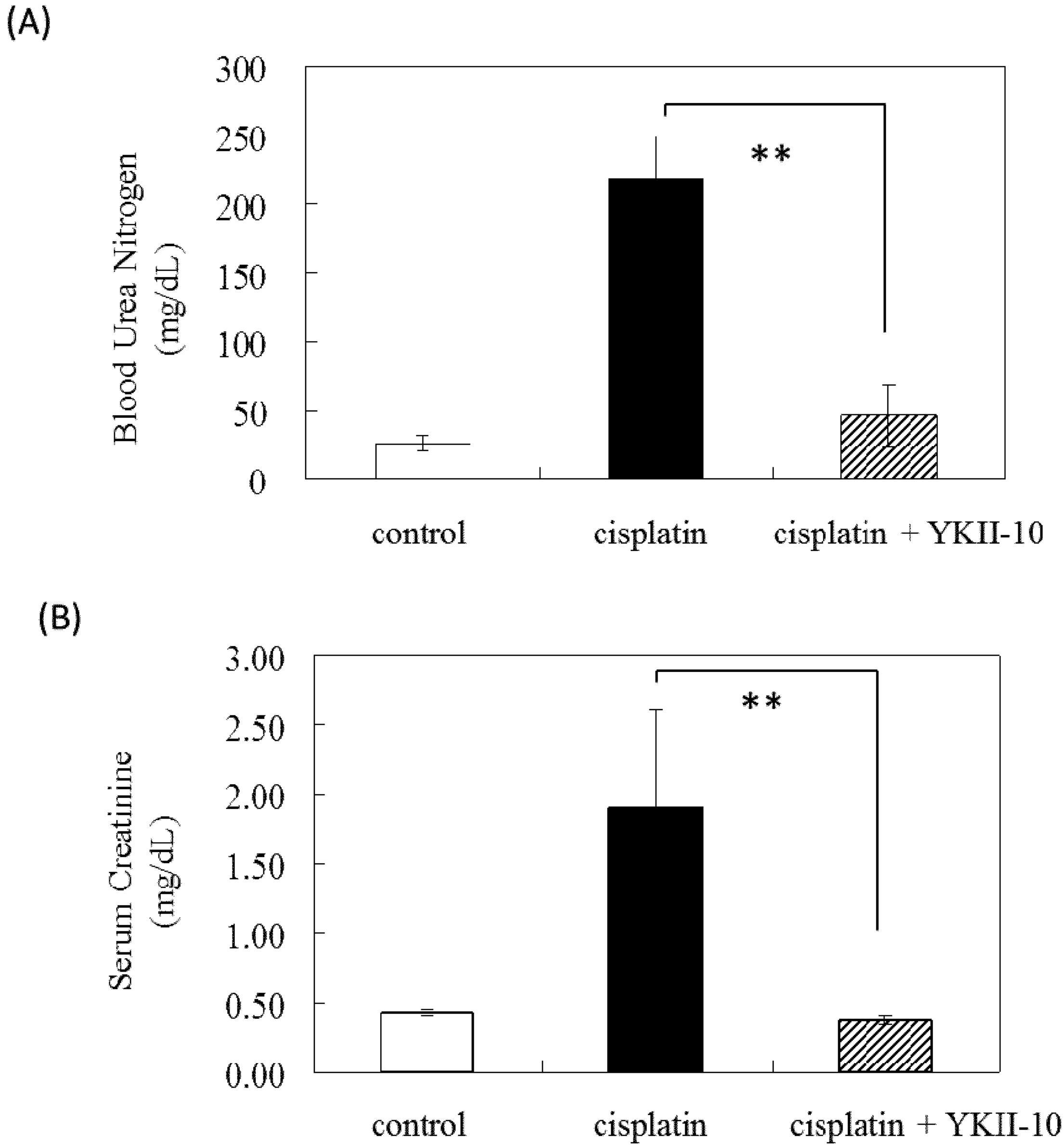
(A)
300
250
200
150
100
50
0
Blood Urea Nitrogen (mg/dL)
**
control
cisplatin
cisplatin + YKII-10
(B)
3.00
2.50
2.00
1.50
1.00
0.50
0.00
Serum Creatinine (mg/dL)
**
control
cisplatin
cisplatin + YKII-10

FIG 2

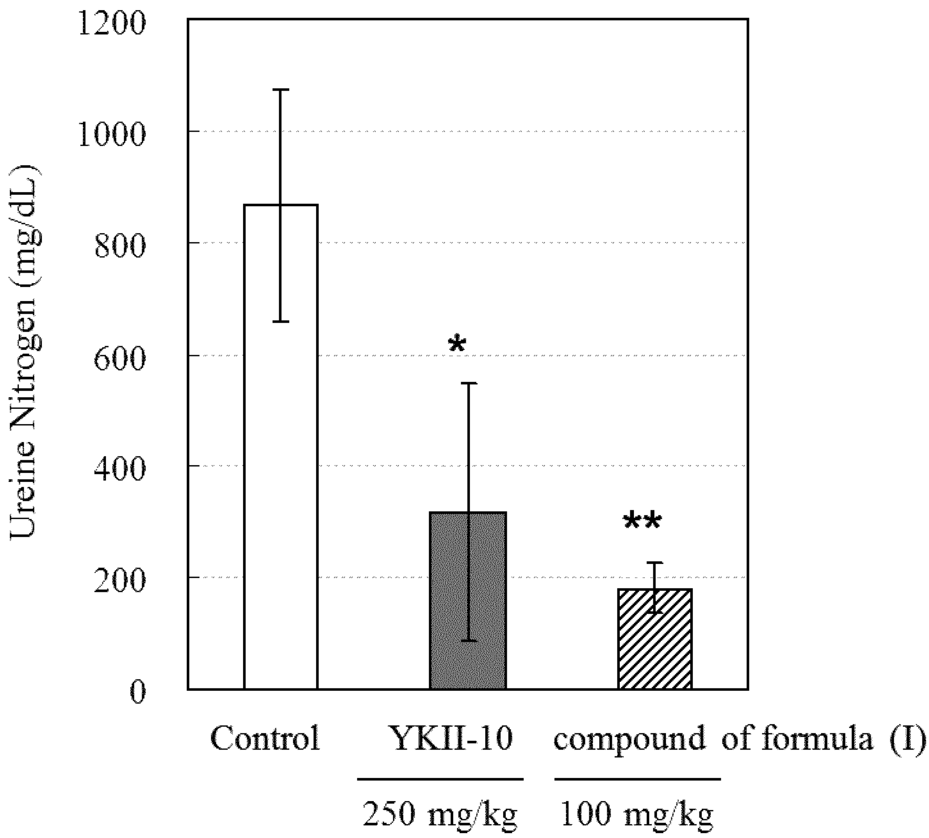
1200
1000
800
600
400
200
0
Ureine Nitrogen (mg/dL)
*
**
Control
YKII-10
compound of formula (I)
250 mg/kg
100 mg/kg

FIG 3

(A)

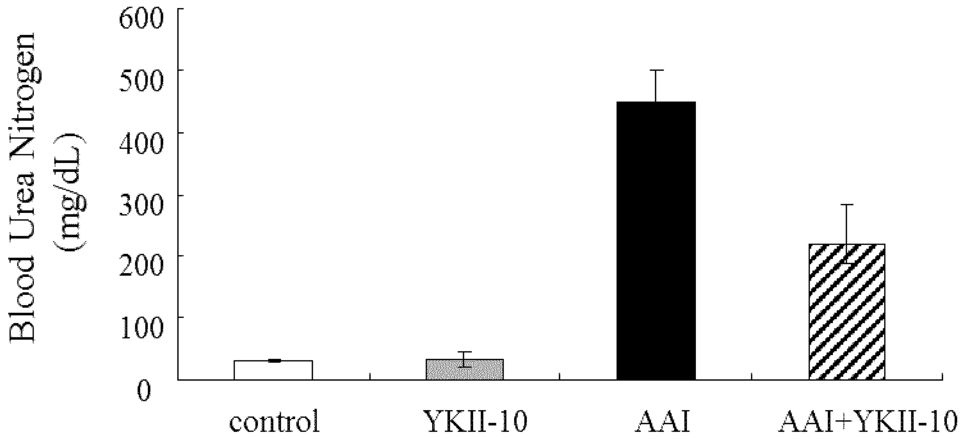
Blood Urea Nitrogen (mg/dL)
600
500
400
300
200
100
0
control
YKII-10
AAI
AAI+YKII-10

(B)

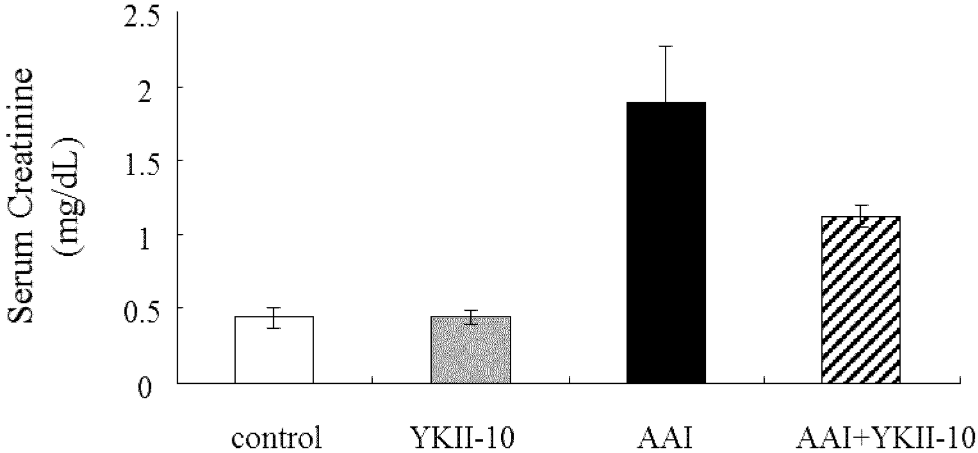
Serum Creatinine (mg/dL)
2.5
2
1.5
1
0.5
0
control
YKII-10
AAI
AAI+YKII-10

FIG 4

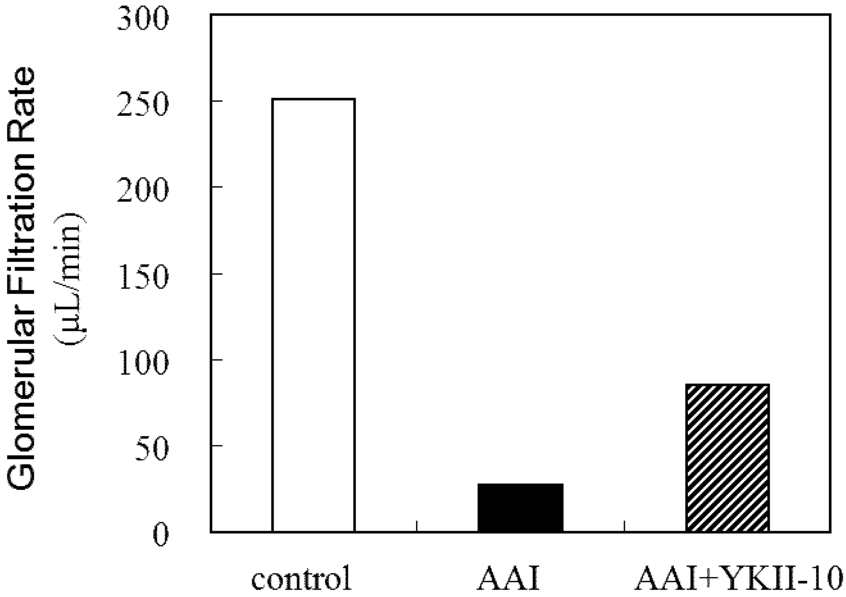
Glomerular Filtration Rate
(μL/min)
300
250
200
150
100
50
0
control
AAI
AAI+YKII-10

FIG 5

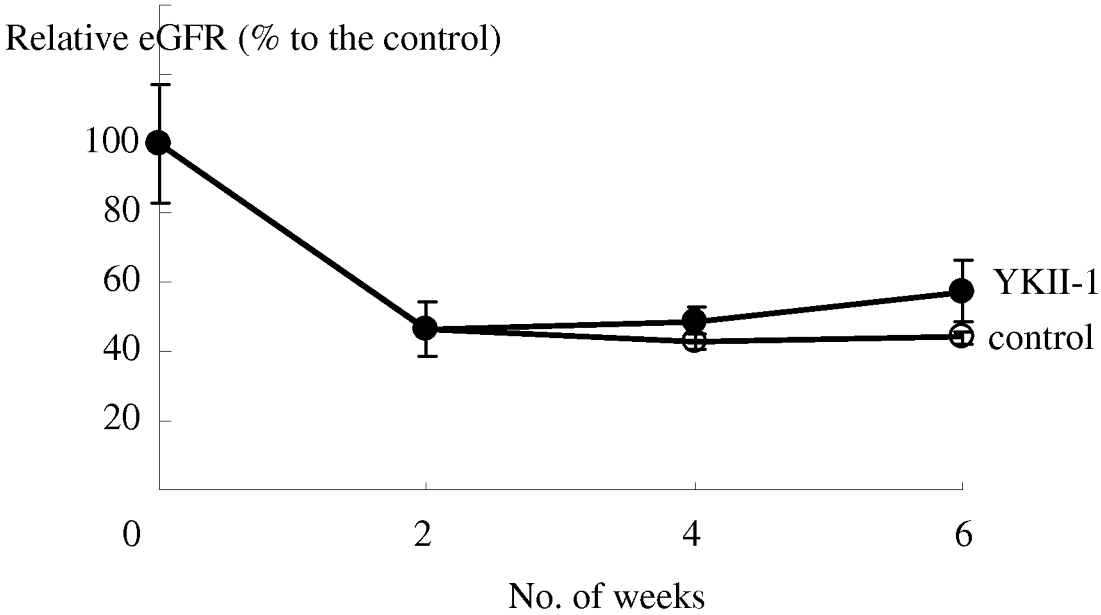
Relative eGFR (% to the control)
100
80
60
40
20
0
2
4
6
No. of weeks
YKII-1
control

FIG 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Exp. Toxicol. Pathol.,* 2011, vol. 63, 157-160
- *Amala Res. Bull.,* 2006, vol. 26, 152-158
- Remington's Pharmaceutical Sciences. Mack Publishing Company, 1985